(19) Europäisches Patentamt
European Patent Office
Office européen des brevets

(11) Publication number: 0 133 331
B1

(12) # EUROPEAN PATENT SPECIFICATION

(45) Date of publication of the patent specification: 05.11.86

(51) Int. Cl.⁴: C 07 C 51/12, C 07 C 53/08, C 07 C 67/36, C 07 C 69/14

(21) Application number: 84200966.4

(22) Date of filling: 03.07.84

(54) **Process for the preparation of acetic acid and/or methyl acetate.**

(30) Priority: 26.07.83 NL 8302644

(43) Date of publication of application: 20.02.85 Bulletin 85/8

(45) Publication of the grant of the patent: 05.11.86 Bulletin 86/45

(84) Designated Contracting States: BE DE FR GB NL

(56) References cited:
EP-A-0 048 046
EP-A-0 090 443
GB-A-1 306 863

(73) Proprietor: SHELL INTERNATIONALE RESEARCH MAATSCHAPPIJ B.V., Carel van Bylandtlaan 30, NL-2596 HR Den Haag (NL)

(72) Inventor: Van Leeuwen, Petrus Wilhelmus Nicolaas Maria, Badhuisweg 3, NL-1031 CM Amsterdam (NL)
Inventor: Roobeek, Cornelis Franciscus, Badhuisweg 3, NL-1031 CM Amsterdam (NL)

(74) Representative: Aalbers, Onno, P.O. Box 302, NL-2501 CH The Hague (NL)

EP 0 133 331 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1986

**Description**

The invention relates to a process for the preparation of acetic acid and/or methyl acetate by reaction of methanol with carbon monoxide in the presence of a catalyst system comprising a palladium compound which is soluble in the reaction mixture and a compound RZ in which R represents hydrogen or an alkyl, cycloalkyl or aralkyl group and Z represents chlorine, bromine or iodine.

From German Auslegeschrift 1 115 234 it is known that acetic acid or a mixture of acetic acid and methyl acetate can be obtained by reaction of methanol with carbon monoxide in the presence of a cobalt catalyst and an iodine compound. A drawback to this process is that it requires the use of a relatively high reaction temperature (230°C) and a high presence (642 bar). A process in which the reaction proceeds at lower temperatures and pressures by using a rhodium catalyst in the presence of an iodine compound is disclosed in UK patent specification 1 233 121. However, rhodium is costly, and the need is felt to have s process which allows practicable results to be achieved by less expensive catalysts.

Netherlands patent specification 6804847 discloses the preparation of carboxylix acids by reacting alcohols with carbon monoxide at a temperature of at least 50°C in the presence of an iridium, platinum, palladium, osmium and/or ruthenium containing catalyst and bromine or iodine or a bromine or iodine compound as promoter. In most of the examples use is made of a catalyst containing iridium, which is about as costly as rhodium. In Example 24 a complex of the less costly metal palladium, [(pC4H9)3P]2PdI2, is used with methyl iodide as promoter. However, the rate at which in the process according to Netherlands patent specification 6804847 carboxylic acids are formed, in particular when using a compound of palladium as catalyst, is unsatisfactory. In Example 24 the rate of acetic acid formation is about 7.4 mol/mol Pd/h at 175°C and at an iodine concentration in the reaction mixture of 0.9 gram atom/l.

Our co-pending European patent application 83200336.2 discloses a process for the preparation of acetic acid and/or methyl acetate by reaction of methanol with carbon monoxide in the presence of a catalytic quantity of a soluble compound of a Group VIII metal, in particular palladium, a promoter RZ, wherein R represents a C1-C3 alkyl group or H and Z is chlorine, bromine or iodine, and iodide or a carboxylate of a Group IA or IIA metal or of a transition metal, and one or more ligands containing at least one electron-rich nitrogen atom. In this process the carbonylation of methanol proceeds at a higher rate than in the process of Netherlands patent application 6804847. It is an essential requirement of the process of the European patent application mentioned that the reaction be carried out in the presence of a ligand which contains at least one electron-rich nitrogen atom.

A process has now been found in which the presence of such a ligand in the reaction mixture is not required, but in which, nevertheless, the desired carbonylation of methanol proceeds at a comparably high rate. The invention relates to a process for the preparation of acetic acid and/or methyl acetate by reaction of methanol with carbon monoxide in the presence of a catalyst system containing a palladium compound which is soluble in the reaction mixture and a compound RZ, wherein R represents hydrogen or an alkyl, cycloalkyl or aralkyl group and Z is chlorine, bromine or iodine, characterized in that, in addition, there are present in the catalyst system an ionic bromine or iodine compound other than HBr or HI and a sulphone or sulphoxide.

Suitable palladium compounds which can be used in the process of the invention are for instance salts, such as palladium chloride, palladium bromide, palladium iodide, palladium nitrate, palladium, sulphate, palladium acetate, palladium propionate and palladium isobutyrate and complexes such as palladiumacetyl acetonate, sodium tetrachloropalladate, potassium tetrachloropalladate, potassium tetraiodopalladate, $[Pd(CO)C1_2]_2$, $Pd[(C_6H_5)_3P]_2I_2$, $Pd[(n\text{-}C_4H_9)_4P]_2C_{14}$, $Pd[(C_6H_5)_3P]_2(C0)Br_2$ and $Pd[(n\text{-}C_4H_9)_3P]_2I_2$. Palladium acetate is particularly preferred.

The palladium compound is preferably used in a concentration of $10^{-5}$-$10^{-1}$ gram atom palladium per litre reaction mixture. In addition to the palladium-containing catalyst, a co-catalyst may be present in the catalyst system, if desired. Nickel compounds, in particular nickel salts, such for instance as nickel chloride and nickel acetate, are suitable co-catalysts.

The ratio of the number of moles of the ionic bromine or iodine compound to the number of gram atoms of palladium generally lies between 1000:1 and 2:1, preferably between 600:1 and 10:1, in particular between 400:1 and 10:1. The ratio of the number of moles of the compound RZ to the number of gram atoms of palladium preferably lies between 2000:1 and 1:1, in particular between 1000:1 and 10:1.

In the compound RZ preference is given to iodine for Z. If R is an alkyl group it preferably has 1-3 carbon atoms. Methyl iodide is an RZ compound used by preference.

Suitable ionic bromine or iodine compounds are the bromides or iodides of the alkali metals, such for instance as lithium, sodium, potassium, rubidium or cesium, of the metals of Group II, such for instance as magnesium, calcium, strontium, barium and zinc, and of the non-noble transition metals of the Group VII and VIII, such for instance as iron, cobalt, nickel and manganese. Quaternary ammonium and phosphonium compounds $R^1_4NX$ and $R^1_4PX$, wherein the four $R^1$ groups independently represent hydrogen or an alkyl of aryl group and X is bromine or iodine, are also suitable. The alkyl groups preferably have 1-6 carbon atoms. Examples of suitable quaternary ammonium or phosphonium compounds are tetramethylammonium iodide, tetraethylammonium bromide, tetraethylammonium iodide, tetrabutylammonium iodide, tetrabutylphosphonium bromide, tetrabutylphosphonium iodide, triphenylphosphonium iodide and methyltriphenylphosphonium iodide.

The sulphone may be represented by the general formula:

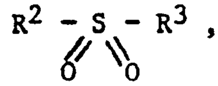

wherein $R^2$ and $R^3$ independently represent an aliphatic or aromatic group, and, when $R^2$ and $R_3$ are aliphatic groups, they may, together with the sulphur atom, form a heterocyclic ring. Preferably the groups $R^2$ and $R^3$ independently represent alkyl groups having 1-12 carbon atoms, or aryl groups, in particular phenyl groups. Specific examples of such sulphones are dimethyl, diisopropyl, dibutyl, methylethyl, methylbutyl and phenylbutyl sulphone.

Examples of preferred sulphones of the general formula given hereinbefore, wherein $R^2$ and $R^3$ together with the sulphur atom form a heterocyclic ring, are sulpholane and the alkyl sulpholanes. The alkyl sulpholanes contain one or more alkyl groups preferably having 1-8 carbon atoms. Specific examples are 2-methylsulpholane, 3-methylsulpholane, 3-butylsulpholane, 3-isopropylsulpholane and 2-methyl-4-butylsulpholane.

The sulphoxide may be represented by the general formula:

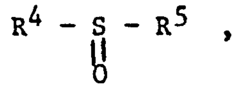

wherein $R^4$ and $R^5$ are similar or dissimilar alkyl groups having not more than 12 carbon atoms. Specific examples of suitable sulphoxides are dimethyl sulphoxide and diethyl sulphoxide.

The molar ratio of the quantity of sulphone or sulphoxide to the quantity of methanol generally lies between 0.05:1 and 20:1, preferably between 0.5:1 and 10:1 and in particular between 0.5:1 and 5:1.

The reaction may optionally be carried out in the presence of a secondary or tertiary phosphine oxide or a phosphinic acid as promoter. Examples of suitable phosphine oxides are trimethylphosphine oxide, diethylphosphine oxide, tri-n-butylphosphine oxide, trioctylphosphine oxide, diphenylphosphine oxide, tri-p-tolylphosphine oxide, tricyclohexylphosphine oxide, diphenylethylphosphine oxide, tri(l-naphthyl)-phosphine oxide and tri-4-chlorophenylphosphine oxide. The phosphorus atom of the phosphine oxide may also be part of a heterocyclic system, such for instance as in 1-phenylphospholane oxide, 1-phenylphosphorinane oxide and 9-phenyl-9-phosphabi-cyclo[3.3.1]nonane oxide. Oxides of phosphines having two or more phosphine groups, such for instance as tetraphenyldiphosphine ethane, are also suitable. Triphenylphosphine oxide and diphenylphosphine oxide, are preferred phosphine oxides. Suitable phosphinic acids are for instance dimethylposphinic acid, dibutylphosphinic acid and in particular diphenylphosphinic acid.

The reaction is generally carried out at a temperature between 125 and 250°C, preferably between 165 and 225°C and in particular between 170 and 205°C. The carbon monoxide partial pressure generally lies between 1 and 360 bar, preferably between 20 and 210 bar and in particular between 20 and 120 bar. High pressures, for instance of up to 1000 bar, may be used, if desired, but in general they are unattractive for technical and economic reasons.

The carbon monoxide used in the process according to the invention may optionally be mixed with inert gases, such for instance as carbon dioxide, methane, nitrogen or noble gases. Hydrogen may also be present; however, the hydrogen content of the carbon monoxidecontaining gas is preferably lower than 50%, in particular lower than 5%.

The process is carried out in the liquid phase. There is usually no need for the use of a(n additional) solvent, since the methanol, the sulphone or sulphoxide and the products formed act sufficiently as solvent. If desired, additional amounts of these compounds can be added to the reaction mixture. Suitable additional solvents are butyrolactone, dimethyl ether, diethyl ether, acetic acid anhydride, methyl-t-butyl ether, alkyl ethers of polyethylene glycols, such as diglyme and tetraglyme, tetrahydrofuran, 1,3-dioxane and 1,4-dioxane. Water may also be present; this will generally stimulate the formation of acetic acid in relation to methyl acetate.

The process of the invention can be carried out continuously, semi-continuously or batch-wise. The reaction mixture obtained can be processed by known techniques, such for instance as fractional distillation. The process can also be integrated into existing processes for the preparation of the starting materials or for further processing of the acetic acid and/or methyl acetate obtained.

**Example 1**

Into a magnetically stirred 100 ml Hastelloy C autoclave (Hastelloy is a trade mark) were placed 10 ml methanol, 35 ml sulpholane, 2.13 g methyl iodide (15 mmol) and the amounts given in Table A of $Pd(CH_3COO)_2$, NaI and, optionally, $Ni(CH_3COO)_2.4H_2O$. The autoclave was flushed with carbon monoxide, filled with carbon monoxide at a pressure of 40 bar, heated in a heater unit to 175°C in 60 min time, and kept at that temperature for 1 hour. The conversion realised during the heating-up period is equal to the conversion obtained during 15 min at reaction temperature, so that the effective reaction time is 1.25 h. Subsequently the autoclave was removed from the heater unit and rapidly cooled in ice. The contents of the autoclave were analysed by gas-liquid chromatography. The quantities of acetic acid and methyl acetate were determined and the total quantity of acetic acid formed by carbonylation of methanol was calculated. The carbonylation rate expressed as mol acetic acid/gram atom Pd/h is given in the table.

**Table A**

| Exp. No. | $Pd(CH_3COO)_2$ (mmol) | NaI (mmol) | $Ni(CH_3COO)_2 \cdot 4H_2O$ (mmol) | mol acetic acid/gram atom Pd/h |
|---|---|---|---|---|
| 1* | 0.04 | 15 | — | 415 |
| 2 | 0.08 | 15 | — | 415 |
| 3 | 0.08 | 17 | 0.68 | 470 |
| 4 | 0.16 | 30 | — | 460 |
| 5 | — | 17 | 0.68 | <10 (calculated per gram atom Ni) |

* In this experiment the effective reaction time was 1.75 h.

Experiment 5, as compared with Experiments 1-4, shows that the presence of palladium is essential for achieving a high reaction rate. It is also seen that by using the palladium-containing catalyst system of the invention very much higher reaction rates are achieved than with the palladium-containing catalyst system used in Netherlands patent application 6804847. For instance, in Experiment 2 of Table A the formation rate is 415 mol acetic acid/gram atom Pd/h at an iodine concentration of 0.65 mol/l, whereas in Example 24 of Netherlands patent application 6804847 acetic acid was formed at a rate of 7.4 mol/mol Pd/h at an iodine concentration of 0.9 mol/l.

**Example II**

Into a magnetically stirred 100 ml Hastelloy C autoclave (Hastelloy is a trade mark) were placed 10 ml methanol, 20 ml sulpholane, 2.75 ml $CH_3I$ (44 mmol) and the amounts given in Table B of $Pd(CH_3COO)2$ and CsI, LiI, tetramethylammonium iodide, triphenylphosphine or diphenylphosphine oxide. From triphenylphosphine, methyltriphenylphosphonium iodide is formed in situ and from diphenylphosphine oxide, dimethyldiphenylphosphonium iodide and diphenylphosphinic acid, which compounds act as ionic iodine compound and promoter, respectively. Furthermore, in some experiments triphenylphosphine oxide or diphenylphosphinic acid was added as promoter. The autoclave was flushed with carbon monoxide, filled with carbon monoxide at a pressure of 40 bar, heated in a heater unit to 175°C or 180°C in 60 min and kept at that temperature for certain periods of time. The conversion achieved during the warmingup period is equal to the conversion achieved during 15 min at reaction temperature. Table B gives total reaction times, i.e. the time during which the reaction mixture was kept at the reaction temperature, plus the effective reaction time (15 min) of the warming-up period. Subsequently the autoclave was removed from the heater unit and rapidly cooled in ice. The contents of the autoclave were analysed by gas-liquid chromatography. The quantities of acetic acid and methyl acetate were determined and the total amounts of acetic acid formed by carbonylation of methanol were calculated. The carbonylation rates, expressed as mol acetic acid/gram atom Pd/h are given in the table.

**Table B**

| Exp. No. | $Pd(CH_3COO)_2$ (mmol) | Iodine compound (mmol) | Promoter (mmol) | Reaction time (min) | Temp. (°C) | mol acetic acid/gram atom Pd/h |
|---|---|---|---|---|---|---|
| 1 | 0.25 | CsI (1) | — | 75 | 180 | 108 |
| 2 | 0.25 | CsI (3,85) | — | 75 | 180 | 196 |
| 3 | 0.25 | CsI (8) | — | 75 | 180 | 240 |
| 4 | 0.25 | CsI (4) | $(C_6H_5)_3PO$ (0,25) | 120 | 175 | 200 |
| 5 | 0.25 | CsI (4) | $(C_6H_5)_2PO_2H$ (1) | 120 | 175 | 280 |
| 6 | 0.25* | CsI (4) | — | 105 | 175 | 100** |
| 7 | 0.25 | LiI (4) | — | 75 | 180 | 181 |
| 8 | 0.25 | $(CH_3)_4NI$ (5) | — | 60 | 175 | 256 |
| 9 | 0.25 | $P(C_6H_5)_3$ (5) | — | 80 | 175 | 180 |
| 10 | 0.25 | $P(C_6H_5)_2OH$ (4) | — | 120 | 175 | 240 |
| | | | Comparative Examples | | | |
| 11 | 0.5 | — | — | 120 | 175 | 14 |
| 12 | 0.25 | — | $(C_6H_5)_3PO$ (8) | 120 | 175 | 40 |
| 13 | 0.25*** | CsI (4) | — | 75 | 180 | <2 |

* A $H_2$ and CO mixture (molar ratio 1:1) was used instead of CO
** By-product acetaldehyde
*** $(C_6H_5CN)_2PtCl_2$ instead of $Pd(CH_3COO)_2$.

When Experiment 2 was repeated using not 3.85, but 4 mmol CsI and a mixture of 20 ml ethyl acetate and 5 ml water instead of 20 ml sulpholane at a reaction temperature of 180°C, no acetic acid or methyl acetate was formed. This experiment shows that the presence of a sulphone in the reaction mixture is essential. Experiments 11 and 12 show that in the absence of an ionic iodide, the reaction rate drops sharply, even in the presence of a promoter (triphenylphosphine oxide). Experiment 13 shows that a catalyst based on the Group VIII metal platinum, which is related to palladium, shows no catalytic action.

## Claims

1. A process for the preparation of acetic acid and/or methyl acetate by reaction of methanol with carbon monoxide in the presence of a catalyst system comprising a palladium compound which is soluble in the reaction mixture and a compound RZ, wherein R represents hydrogen or an alkyl, cycloalkyl or aralkyl group, and Z is chlorine, bromine or iodine, characterized in that in addition there are present in the catalyst system an ionic bromine or iodine compound other than HBr or HI and a sulphone or sulphoxide.

2. A process as claimed in claim 1, characterized in that, in addition to the palladium compound, a nickel compound is present in the catalyst system.

3. A process as claimed in claim 1 or 2, characterized in that the ratio of the number of moles of the ionic bromine or iodine compound to the number of gram atoms palladium lies between 1000:1 and 2:1, in particular between 600:1 and 10:1.

4. A process as claimed in claims 1-3, characterized in that the ionic bromine or iodine compound is a bromide or iodide of an alkali metal, a Group II metal or of a non-noble transition metal of Group VII or VIII, or a quaternary ammonium or phosphonium compound $R^1_4NX$ or $R^1_4PX$, wherein the four groups $R^1$ independently represent hydrogén or an alkyl or aryl group, and X is bromine or iodine.

5. A process as claimed in claims 1-4, characterized in that the sulphone has the general formula

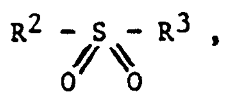

wherein $R^2$ and $R^3$ independently represent an aliphatic or aromatic group, and, when $R^2$ and $R^3$ are aliphatic groups, they may, together with the sulphur atom, form a heterocyclic ring.

6. A process as claimed in claim 5, characterized in that the groups $R^2$ and $R^3$ independently represent alkyl groups having 1-12 carbon atoms, or aryl groups, in particular phenyl groups.

7. A process as claimed in claim 5, characterized in that the sulphone is sulpholane or an alkyl sulpholane.

8. A process as claimed in claims 1-4, characterized in that the sulphoxide has the general formula

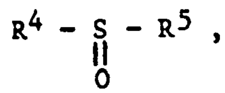

wherein $R^4$ and $R^5$ are similar or dissimilar alkyl groups having not more than 12 carbon atoms.

9. A process as claimed in claims 1-8, characterized in that the molar ratio of the quantity of sulphone or sulphoxide to the quantity of methanol lies between 0.05:1 and 20:1, in particular between 0.5:1 and 10:1.

10. A process as claimed in claims 1-9, characterized in that the reaction is carried out in the presence of a secondary or tertiary phosphine oxide or a phosphinic acid as promoter.

## Patentansprüche

1. Verfahren zur Herstellung von Essigsäure und/oder Methylacetat durch Umsetzung von Methanol mit Kohlenmonoxid in Gegenwart eines Katalysatorsystems umfassend eine Palladiumverbindung, die in dem Reaktionsgemisch löslich ist,und eine Verbindung RZ, in der R ein Wasserstoffatom oder eine Alkyl-, Cycloalkyloder Aralkylgruppe bedeutet und Z eine Chlor-, Brom- oder Jodatom ist, dadurch gekennzeichnet, daß zusätzlich in dem Katalysatorsystem eine andere ionische Brom- oder Jodverbindung vorhanden ist als HBr- oder HJ und ein Sulfon oder Sulfoxid.

2. Verfahren nach Anspruch 1, dadurch gekennzeichnet, daß zusätzlich zu der Palladiumverbindung eine

Nickelverbindung in dem Katalysatorsystem vorhanden ist.

3. Verfahren nach Anspruch 1 oder 2, dadurch gekennzeichnet, daß das Verhältnis der Anzahl von Molen der ionischen Brom- oder Jodverbindung zu der Anzahl g-Atom Palladium zwischen 1 000: 1 und 2: 1, insbesondere zwischen 600: 1 und 10: 1, liegt.

4. Verfahren nach Anspruch 1 bis 3, dadurch gekennzeichnet, daß die ionische Brom- oder Jodverbindung ein Bromid oder Jodid eines Alkalimetalls, eines Metalls der Gruppe II oder eines nicht-edlen Übergangsmetalls der Gruppe VII oder VIII oder eine quaternäre Ammonium- oder Phosphoniumverbindung $R^1{}_4NX$ oder $R^1{}_4PX$ ist, wobei die vier Gruppen $R^1$ unabhängig voneinander ein Wasserstoffatom oder eine Alkyl- oder Arylgruppe bedeuten und X Brom oder Jod ist.

5. Verfahren nach Anspruch 1 bis 4, dadurch gekennzeichnet, daß das Sulfon der folgenden allgemeinen Formel entspricht

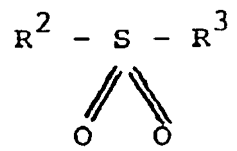 ,

in der $R^2$ und $R^3$ unabhängig voneinander eine aliphatische oder aromatische Gruppe bedeuten und wenn $R^2$ und $R^3$ aliphatische Gruppen sind, diese zusammen mit dem Schwefelatom einen heterocyclischen Ring bilden können.

6. Verfahren nach Anspruch 5, dadurch gekennzeichnet, daß die Gruppen $R^2$ und $R^3$ unabhängig voneinander Alkylgruppen mit 1 bis 12 Kohlenstoffatomen oder Arylgruppen, insbesondere Phenylgruppen, bedeuten.

7. Verfahren nach Anspruch 5, dadurch gekennzeichnet, daß das Sulfon Sulfolan oder ein Alkylsulfolan ist.

8. Verfahren nach Anspruch 1 bis 4, dadurch gekennzeichnet, daß das Sulfoxid die folgende allgemeine Formel hat

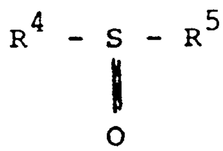 ,

in der $R^4$ und $R^5$ gleiche oder verschiedene Alkylgruppen mit nicht mehr als 12 Kohlenstoffatomen sind.

9. Verfahren nach Anspruch 1 bis 8, dadurch gekennzeichnet, daß das Molverhältnis der Menge an Sulfon oder Sulfoxid zu der Menge an Methanol zwischen 0,05: 1 und 20: 1, insbesondere zwischen 0,5: 1 und 10: 1, liegt.

10. Verfahren nach Anspruch 1 bis 9, dadurch gekennzeichnet, daß die Reaktion in Gegenwart eines sekundären oder tertiären Phosphinoxids oder einer Phosphonsäure als Beschleuniger durchgeführt wird.

**Revendications**

1. Procédé de préparation d'acide acétique et/ou d'acétata da méthyle par réaction de méthanol avec du monoxyde de carbone en présence d'un système catalyseur comprenant un composé de palladium qui est soluble dans la mélange réactionnel at un composé RZ, où R représente un hydrogène ou un groupe alcoyle, cycloalcoyle ou aralcoyle, et Z est un chlore, un brome ou un iode, caractérisé en ce que en addition se trouve dans le système catalysaur un composé ionique de brome ou d'iode autre qu'HBr ou HI et une sulfone ou un sulfoxyde.

2. Procédé selon la revendication 1, caractérisé en ce que, outre le composé de palladium, un composé de nickel est présent dans le système catalyseur.

3. Procédé selon les revendications 1 et 2, caractérisé en ce que le rapport du nombre de moles du composé ionique de brome ou d'iode au nombre d'atomes-grammes de palladium se situe entre 1000:1 et 2:1, en particulier entre 600:1 et 10:1.

4. Procédé selon l'une des revendications 1 à 3, caractérisé en ce que le composé ionique de brome ou d'iode est un bromure ou iodure d'un métal alcalin, d'un métal du groupe II ou d'un métal de transition non noble du groupe VII ou VIII, ou un composé d'ammonium ou de phosphoniun quaternaire $R^1{}_4$ NX ou $R^1{}_4$ PX, où les quatre groupes $R^1$ représentent indépendamment un hydrogène ou un groupe alcoylé ou aryle, et X est un brome ou un iode.

5. Procédé selon l'une des revendications 1 à 4, caractérisé en ce que la sulfone a la formule générale

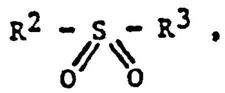

où $R^2$ et $R^3$ représentent indépendamment un groupe aliphatique ou aromatique, et où lorsque R2 et R3 sont des groupes aliphatiques, ils peuvent, ensemble avec l'atome de soufre, former un noyau hétérocyclique.

6. Procédé selon la revendication 5, caractérisé en ce que les groupes R2 et R3 représentent indépendamment des groupes alcoyle ayant de 1 à 12 atomes de carbone, ou des groupes aryle, en particulier des groupes phényle.

7. Procédé selon la revendication 5, caractérisé en ce que la sulfone est le sulfolane ou un alcoyl-sulfolane.

8. Procédé selon l'une des revendications 1 à 4, caractérisé en ce que le sulfoxyde a la formule générale

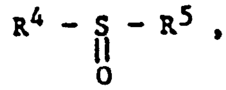

où R4 et R5 sont des groupes alcoyle semblables ou différents n'ayant pas plus de 12 atomes de carbone.

9. Procédé selon les revendications 1-8, caractérisé en ce que le rapport molaire de la quantité de sulfone ou de sulfoxyde à la quantité de méthanol se situe entre 0,05:1 et 20:1, en particulier entre 0,5:1 et 10:1.

10. Procédé selon l'une des revendications 1 à 9, caractérisé en ce que la réaction est conduite en présence d'un phosphine-oxyde secondaire ou tertiaire ou d'un acide phosphinique comme promoteur.